Europäisches Patentamt

European Patent Office

Office européen des brevets

(19)

(11) Veröffentlichungsnummer. **0 010 136 B1**

(12) **EUROPÄISCHE PATENTSCHRIFT**

(45) Veröffentlichungstag der Patentschrift:
02.03.83

(51) Int. Cl.³: **A 61 B 5/00,** A 61 B 5/05 //
G01N27/48, G01N27/30

(21) Anmeldenummer: 79103088.5

(22) Anmeldetag: 22.08.79

(54) **Messwertaufnehmer, insbesondere zur Bestimmung des Partialdruckes von gelösten Gasen.**

(30) Priorität: **23.08.78 DE 2836868**

(73) Patentinhaber: **SIEMENS AKTIENGESELLSCHAFT,
Berlin und München Wittelsbacherplatz 2,
D-8000 München 2 (DE)**

(43) Veröffentlichungstag der Anmeldung:
**30.04.80 Patentblatt 80/9**

(72) Erfinder: **Reichenberger, Helmut, Dr. Dipl.-Phys.,
Irisstrasse 8, D-8501 Brand/Eckental (DE)**
Erfinder: **Zachmann, Wilfried, Nöttinger Strasse 41,
D-7537 Remchingen (DE)**

(45) Bekanntmachung des Hinweises auf die Patenterteilung:
**02.03.83 Patentblatt 83/9**

(84) Benannte Vertragsstaaten:
**AT BE CH FR GB IT**

(56) Entgegenhaltungen:
**DD-A-48 081
DE-A-2 145 400
DE-A-2 365 477
DE-A-2 640 987
DE-A-2 758 413
DE-B-2 006 682
DE-B-2 255 879
US-A-3 432 418
US-A-3 504 664
US-A-3 998 212
Biomedizinische Technik, Band 19, 1974, Nr. 3, R. Huch et al. »Die kontinuierliche Kontrolle des arteriellen PO2 und ihre Bedeutung für die Überwachung von Mutter und Kind« Seiten 87 bis 91**

## Meßwertaufnehmer, insbesondere zur Bestimmung des Partialdruckes von gelösten Gasen

Die Erfindung bezieht sich auf einen Meßwertaufnehmer, insbesondere zur Bestimmung des Partialdruckes von gelösten Gasen, z. B. transkutane Sauerstoffelektrode, bestehend aus einem Trägerteil für wenigstens einen Edelmetalldraht als Meßelektrode und eine ringförmige Referenzelektrode, wobei sich die Meßelektrode in der Öffnung der ringförmigen Referenzelektrode befindet, sowie für ein Element zum Erwärmen des Meßwertaufnehmers, wobei dieses Element gleichermaßen die Funktion einer Heizung und eines Temperaturfühlers erfüllt.

Es sind Elektrodenanordnungen bekannt, mit denen elektrochemisch der Partialdruck von gelösten Gasen bestimmt wird, beispielsweise der Sauerstoff-Partialdruck mit einem polarographischen Verfahren. Soll nun mit einer solchen Elektrodenanordnung der Sauerstoff-Partialdruck transkutan, also durch die Hautoberfläche eines Lebewesens hindurch, gemessen werden, so muß die Haut zur Messung aktiv hyperämisiert werden. Dazu wird im allgemeinen der Meßwertaufnehmer auf eine gegenüber Körpertemperatur erhöhte Temperatur beheizt. Aus der DE-A-2 145 400 ist eine Anordnung für derartige transkutane Messungen bekannt. Bei dieser wird als Meßelektrode (Kathode) ein Massiver Kupferblock verwendet, der stirnseitig mit Platin beschichtet ist und um den eine ringförmige Bezugselektrode (Anode) aus Silber angeordnet ist. Kathode und Anode sind mit einem Elektrolytfilm bedeckt und gemeinsam mit einer Membran überzogen, die als Auflagefläche der gesamten Anordnung auf der Haut anliegt. Die Kathode steht dabei in thermischem Kontakt mit einem Temperaturfühler und einer Heizeinrichtung, mittels der die Meßelektrode auf eine gegenüber Körpertemperatur höhere Temperatur zwecks Hyperämisierung der Haut aufgeheizt wird. Eine solche Anordnung, in der Heiz- und Temperaturmeßelement separat angeordnet sind, ist mechanisch und elektrisch aufwendig. Jedes im Aufnehmer angeordnete Element benötigt separate Zuleitungen, wodurch erfahrungsgemäß die Störanfälligkeit der Verbindung zwischen Abnehmer und zugehörigem Betriebsgerät erhöht wird. Weiterhin müssen beim Heiz- und Temperaturmeßelement Maßnahmen für einen besonders guten Wärmeübergang zur Meßfläche geschaffen werden. Ferner kann es durch die Beschränkung der Heizung auf die Kathode zur Ausbildung von Temperaturgradienten innerhalb der polarographischen Meßzelle kommen.

Weiterhin ist aus der DE-B-2 640 987 eine transkutane Sauerstoffelektrode bekannt, bei der die Beheizung der Haut auf eine erhöhte Temperatur durch eine den Elektroden vorgeschaltete Heizscheibe mit Gasöffnungen erreicht wird. Hierbei wird die Haut unmittelbar beheizt, und es geht nur wenig Heizleistung durch Wärmeleitung verloren. Allerdings ist eine Stabilisierung einer solchen Heizscheibe mit Gasdurchtrittsöffnungen auf konstante Temperatur, die für eine zuverlässige transkutane Messung notwendig ist, problematisch.

Aus der DE-B2-2 255 879 und dem Aufsatz von R. Huch et al. in der Zeitschrift »Biomedizinische Technik«, Band 19, 1974, Nr. 3, Seiten 87 bis 91 sind Meßwertaufnehmer mit ringförmigen Referenzelektroden, in die Heizwicklungen eingelassen sind, bekannt.

Darüber hinaus wird in der US-A-3 998 212 ein Meßwertaufnehmer zur Bestimmung des Sauerstoffpartialdruckes beschrieben, der ein Trägerteil für einen Edelmetalldraht als Meßelektrode und eine ringförmige Referenzelektrode aufweist, wobei sich die Meßelektrode in der Öffnung der ringförmigen Referenzelektrode befindet, und der eine Heizeinrichtung zum Temperieren des Meßwertaufnehmers hat. Speziell bei diesem Meßwertaufnehmer bildet das Heizelement mit einem Ast der Zuleitung zugleich den einen Schenkel eines Thermoelementes, so daß das gesamte Element über die Aufgabe einer Heizung hinaus auch die Funktion eines Temperaturfühlers erfüllen kann. Allerdings wird dabei die Temperatur am Ende des Heizelementes, also vergleichsweise entfernt von Meß- bzw. Referenzelektroden am interessierenden Applikationsort, gemessen. Dies kann aber im Ergebnis bereits undefinierte Meßverhältnisse bewirken.

Der Erfindung liegt daher die Aufgabe zugrunde, einen Meßwertaufnehmer der eingangs genannten Art zu schaffen, der möglichst einfach aufgebaut ist, eine hohe Meßstabilität aufweist und dessen Meßfläche problemlos auf die konstante Temperatur beheizbar ist.

Die Aufgabe ist erfindungsgemäß dadurch gelöst, daß die ringförmige Referenzelektrode speziell topfförmig mit der Öffnung im Topfboden ausgebildet ist, wobei das Element zum Erwärmen des Meßwertaufnehmers unmittelbar auf dem Boden der topfförmigen Referenzelektrode um die Öffnung herum angeordnet ist.

Vorzugsweise weist die topfförmige Elektrode eine kreisförmige Grundfläche mit zentraler Öffnung auf. Das Element zur Heizung und Temperaturmessung ist als Widerstandsstrecke mit definierter Temperaturabhängigkeit ausgebildet. Die Widerstandsstrecke kann durch eine Flachspule aus dünnem Draht, beispielsweise Kupfer, Nickel oder Platin, oder von auf einem Substrat aufgebrachten Widerstandsschichten gebildet sein.

Durch den erfindungsgemäßen Aufbau eines Meßwertaufnehmers wird der Wärmeübergang vom Heizelement zur Meßfläche optimiert und in der gesamten Meßzelle eine konstante, meßbare Temperatur erreicht. Es ergeben sich so auch über längere Zeiten stabile Verhältnisse, so daß ein solcher Meßwertaufnehmer mit reproduzierbaren Meßwerten zur Langzeitüberwachung verwendet werden kann.

Der Meßwertaufnehmer nach der Erfindung ist also sowohl hinsichtlich seines Aufbaus als auch bezüglich seiner Handhabung gegenüber den aus dem Stand der Technik bekannten Aufnehmern wesentlich vereinfacht. Eine separate Temperaturmessung mittels Thermoelement, Thermistor od. dgl. ist bei Verwendung der beheizbaren Widerstandsstrecke nicht mehr notwendig. Als Widerstandsstrecke wird vorzugsweise ein Metalldraht mit einem Widerstand von etwa 100 Ohm gewählt. Bei Verwendung von beispielsweise Platin beträgt die relative Widerstandsänderung mit der Temperatur im Anwendungsbereich $3,85 \cdot 10^{-3}$ pro Grad. Der Absolutwiderstand der Widerstandsstrecke gibt gleichzeitig die am Meßwertaufnehmer vorliegende Temperatur an. Durch Messung des Widerstandes des Heizelements und Vergleich mit einem Sollwert kann also eine Regelgröße für die Heizspannungsversorgung abgeleitet werden.

Der Meßwertaufnehmer nach der Erfindung hat in bevorzugter Ausgestaltung um den eigentlichen Elektrodenteil ein volumenmäßig wesentlich größeres Trägerteil, das applikationsseitig als ringförmige ebene Auflagefläche ausgebildet ist. Der in diesem Ring zentrisch angeordnete Anodentopf weist eine zentrale Ausnehmung auf, in der sich die Kathode befindet. Dabei hat es sich als vorteilhaf erwiesen, den Anodentopf gegenüber der ebenen Ringfläche konvex auszubilden. Wird der Aufnehmer zur transkutanen Sauerstoffmessung verwendet, so wird vor der Messung unter Einschluß eines Elektrolyten eine sauerstoffdurchlässige Membran auf der Elektrodenfläche fixiert. Verwendet man eine Klebevorrichtung mit beidseitigen Klebeflächen, in die die sauerstoffdurchlässige Membran eingelegt bzw. eingeklebt ist, so läßt sich eine solche Membran gleichzeitig mit Anbringen der Klebehalterung fixieren. Durch die konvexe Ausbildung der Anodenfläche ergibt sich dabei eine erwünschte Vorspannung der formelastischen Membran. Der Meßwertaufnehmer wird so zu einer polarographischen Meßzelle komplettiert. Besonders vorteilhaft erweist sich dabei auch bei Verwendung des Meßwertaufnehmers, daß für den gemeinsamen Betrieb von polarographischer Meßzelle und temperaturgeregelter Widerstandsstrecke nur noch drei Zuleitungen für den Anschluß an ein entsprechend ausgelegtes Betriebsgerät notwendig sind.

Weiter Vorteile und Einzlheiten ergeben sich aus der nachfolgenden Figurenbeschreibung eines Ausführungsbeispiels anhand der Zeichnung in Kombination mit den weiteren Unteransprüchen. Es zeigt

Fig. 1 einen Querschnitt eines kompletten, applizierten Meßwertaufnehmers nach der Erfindung und

Fig. 2 eine schematische Aufsicht auf den Aufnehmer nach Fig. 1,

Fig. 3 eine schematische Aufsicht auf die zugehörige Klebehalterung mit sauerstoffdurchlässiger Membran und

Fig. 4 eine in den Meßwertaufnehmer einsetzbare Heizscheibe in vergrößerter Darstellung.

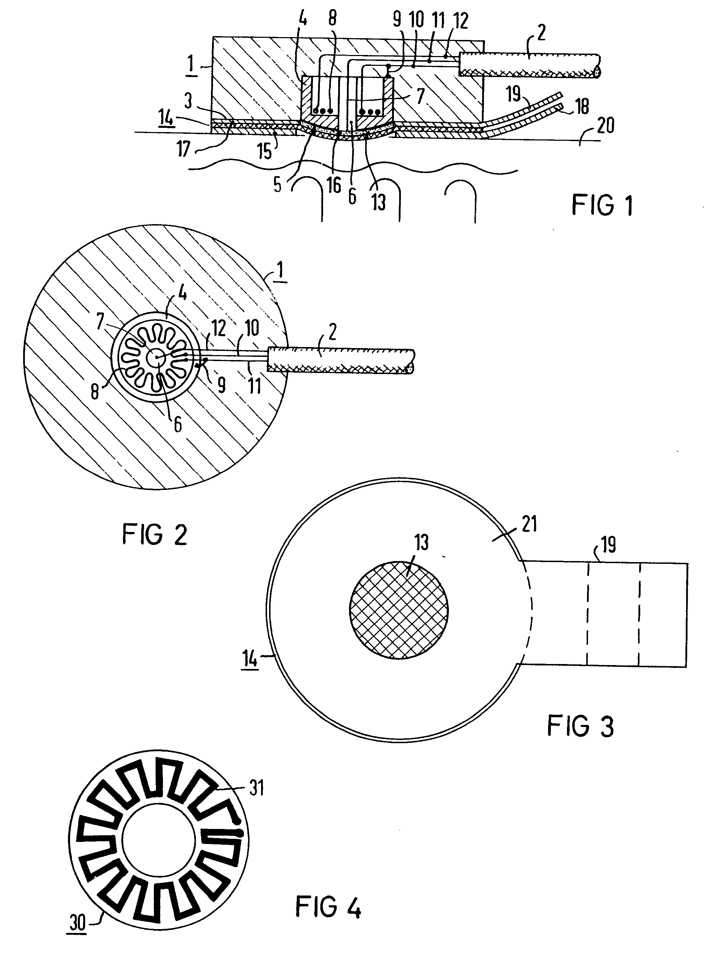

In der Fig. 1 ist mit 1 das Trägerteil eines Meßwertaufnehmers bezeichnet, der auf der Hautoberfläche 20 eines Patienten als Meßort für eine transkutane Sauerstoffmessung aufgesetzt und fixiert ist. Das Trägerteil 1 ist als flache zylindrische Scheibe ausgebildet und hat einen Anschluß 2 mit gleichzeitiger Zugentlastung für elektrische Leitungen. Das Trägerteil 1 besteht gut wärmeisolierenden Kunststoffmaterialien, beispielsweise Epoxydharz. Zur Applikationsseite hin hat das Trägerteil 1 eine ringförmige Auflagefläche 3, die eine genügende Sicherheit gegen Verkippungen, Verschiebungen od. dgl. gewährleistet. Zentral im Trägerteil 1 ist ein Hohlzylindertopf 4 als Anode eingegossen. Dieser Topf 4 besteht aus Silber und ist in geeigneter Weise ausgeformt. Die Zylinderwandung schließt mit ihrer unteren Kante bündig mit der Ringfläche 3 des Trägerteils 1 ab. Die Grundfläche 5 des Topfes 4 ist als Teil einer Kugelfläche ausgebildet, so daß sie gegenüber der Ringfläche 3 eine konvexe Auswölbung bildet. Die konvexe Grundfläche 5 weist in der Mitte eine zentrale Ausnehmung 6 auf, in die ein Platin-Draht 7 von 20 μm isoliert eingesetzt ist. Die polierte Stirnfläche schließt bündig mit konvexen Fläche 5 ab und bildet so die wirksame Kathode.

Auf dem Boden des Anodentopfes 4 sitzt elektrisch isoliert eine Wicklung aus isoliertem Metalldraht, deren äußerer Durchmesser durch die innere Wandung des Anodentopfes 4 und deren innerer Durchmesser durch die Ausnehmung 6 vorgegeben ist. Der zwischen Anodentopf 4, Kathodendraht 7 und Heizwicklung 8 seitlich noch verbleibende Raum ist mit gut wärmeleitendem Material ausgefüllt. Oberhalb der Heizwicklung 8 wird der verbleibende Raum mit thermisch isolierendem Material ausgefüllt. Da das Material des Trägerteils 1, beispielsweise Epoxydharz, nur geringe Wärmeleitfähigkeit hat, wird also praktisch die gesamte in der Heizwicklung 8 erzeugte Wärme zur Elektrodenfläche geleitet. Anode 4, Kathode 7 und Heizwicklung 8 sind über elektrische Zuleitungen 9 bis 12 über den Kabelanschluß 2 mit einem externen Meß- bzw. Heizspannungsversorgungsgerät verbunden. Dabei sind die Leitungen 9 bis 12 zweckmäßigerweise so geschaltet, daß im Anschluß 2 nur drei Einzelleitungen zum Betriebsgerät geführt werden. Die Handhabung des Meßwertaufnehmers ist dadurch besonders unempfindlich gegen Störungen.

Zwischen Elektrodenmeßfläche und Hautoberfläche 20 sind für die Messung ein Elektrolyt 16 sowie eine sauerstoffdurchlässige Membran 13 angeordnet. Zwischen ringförmigem Trägerteil 1 und Haut 20 befindet sich noch zur Langzeitfixierung des gesamten Meßwertaufnehmers am Meßort eine Klebehalterung 14 mit Klebefolien 15 und 17 sowie Anfaßlaschen 18 und 19. Durch die konvexe Auswölbung der Anodenunterfläche 5 wird beim Aufbringen der sauerstoffdurchlässigen Membran 13 und Fixieren mittels Klebehalterung 14 gleichzeitig eine gewisse Vorspannung der formelastischen Membran 13 erzielt. Dadurch ist in einfacher Weise gewährleistet, daß keine Luftblasen im Elektrolyten zwischen Elektrodenunterfläche 5 und Membran 13 verbleiben können.

Die Fig. 2 und 3 verdeutlichen den konzentrischen Aufbau der einzelnen Teile des erfindungsgemäßen Aufnehmers und der zugehörigen Klebehalterung zum Fixieren des Aufnehmers am Meßort. Das Trägerteil 1 mit seiner ebenen Ringfläche und Kabelanschluß 2 umschließt die Anodenfläche 5 mit zentraler Ausnehmung 6. In der Ausnehmung 6 ist punktförmig die wirksame Kathodenfläche 7 angeordnet.

Die zugehörige Klebehalterung 14 nach Fig. 3 besteht aus zwei aufeinanderliegenden Kleberingen (nicht sichtbar) mit Anfaßlaschen 19, zwischen denen die sauerstoffdurchlässige Membran 13 eingelassen ist. In Fig. 3 ist die Klebehalterung 14 durch ein nicht klebendes Abdeckpapier 21, das gleichermaßen die Anfaßlasche 19 umfaßt, abgedeckt.

Im Ausführungsbeispiel wird als heizbares Widerstandselement vorzugsweise eine Platindrahtwicklung verwendet, die einen Gesamtwiderstand von etwa 100 Ohm aufweisen soll. Ein Wert dieser Größenordnung hat sich bei Untersuchungen als zweckmäßig erwiesen, da einerseits die bei Temperaturänderungen auftretenden Widerstandsänderungen noch mit guter Auflösung erfaßt werden können und andererseits die zur Beheizung der Widerstandsstrecke notwendigen Versorgungsspannungen bzw. -ströme in einer solchen Größenordnung liegen, wie sie durch üblicherweise verwendete Bauelemente sowie Vorschriften für biomedizinische Abnehmer vorgegeben sind. Bei einem Widerstandstemperaturkoeffizienten von $3,85 \cdot 10^{-3}$ beträgt dann die Widerstandsänderung etwa 0,4 Ohm und der zur Temperierung des auf der Haut applizierten Abnehmers auf 42°C notwendige Strom etwa 20 bis 100 mA.

Statt Platin können natürlich ebensogut Anordnungen aus anderen Materialien mit geeigneten Widerständen und Temperaturkoeffizienten, beispielsweise Kupfer oder Nickel, als Heizelemente verwendet werden. So können in weiterer Ausbildung der Erfindung als Heizeinrichtung auch Widerstandselemente in Schichttechnologie benutzt werden. Solche Widerstandselemente bestehen beispielsweise aus einem Substrat durch Aufdampfen, elektrolytische Abscheidung od. dgl. aufgebrachten Nickelschichten. Diese Elemente können wegen ihrer besonders flachen Bauweise unmittelbar mit dem Anodentopf in thermischen Kontakt gebracht werden. In Fig. 4 ist ein Substrat mit 30 und eine darauf aufgebrachte Nickelschicht mit 31 bezeichnet. Die als Heizwiderstand wirkende Nickelschicht 31 ist ein geeigneter Weise dabei als Mäanderband ausgebildet. Durch Vorgabe definierter Geometrie und Schichtdicke lassen sich Widerstände in gewünschter Größenordnung herstellen. Auch solche Widerstandselemente in Schichttechnologie lassen sich in geeigneter Weise gleichermaßen als Heizung und Temperaturmeßfühler verwenden.

**Patentansprüche**

1. Meßwertaufnehmer, insbesondere zur Bestimmung des Partialdruckes von gelösten Gasen, z. B. transkutane Sauerstoffelektrode, bestehend aus einem Trägerteil (1) für wenigstens einen Edelmetalldraht als Meßelektrode (7) und eine ringförmige Referenzelektrode (4), wobei sich die Meßelektrode (7) in der Öffnung (6) der ringförmigen Referenzelektrode (4) befindet, sowie für ein Element (8) zum Erwärmen des Meßwertaufnehmers, wobei dieses Element (8) gleichermaßen die Funktion einer Heizung und eines Temperaturfühlers erfüllt, dadurch gekennzeichnet, daß die ringförmige Referenzelektrode (4) speziell topfförmig ist der Öffnung (6) im Topfboden ausgebildet ist, wobei das Element (8) zum Erwärmen des Meßwertaufnehmers unmittelbar auf dem Boden der topfförmigen Referenzelektrode (4) um die Öffnung (6) herum angeordnet ist.

2. Meßwertaufnehmer nach Anspruch 1, dadurch gekennzeichnet, daß die topfförmige Referenzelektrode (4) eine kreisförmige Grundfläche (5) mit zentraler Öffnung (6) aufweist.

3. Meßwertaufnehmer nach Anspruch 1, dadurch gekennzeichnet, daß das Element (8) eine temperaturabhängige Widerstandsstrecke ist.

4. Meßwertaufnehmer nach Anspruch 3, dadurch gekennzeichnet, daß die Widerstandsstrecke einen elektrischen Widerstand in der Größenordnung von 100 Ohm aufweist.

5. Meßwertaufnehmer nach Anspruch 3 oder 4, dadurch gekennzeichnet, daß die Widerstandsstrecke durch eine Flachspule aus dünnem Draht, beispielsweise Kupfer, Nickel oder Platin, gebildet ist.

6. Meßwertaufnehmer nach Anspruch 3 oder 4, dadurch gekennzeichnet, daß die Widerstandsstrecke von auf einem Substrat (30) aufgebrachten Widerstandsschichten (31) gebildet ist.

7. Meßwertaufnehmer nach Anspruch 1, dadurch gekennzeichnet, daß das Trägerteil (1) eine Auflagefläche (3) für eine Klebehalterung (14) zum Fixieren des Meßwertaufnehmers auf der Haut (20) des Meßortes aufweist.

8. Meßwertaufnehmer nach Anspruch 1, dadurch gekennzeichnet, daß die topfförmige Referenzelektrode (4) mit ihrem äußeren Boden (5) konvex zur Auflagefläche (3) des Trägerteils (1) ausgebildet ist.

9. Meßwertaufnehmer nach Anspruch 8, dadurch gekennzeichnet, daß die Auflagefläche (3) ringförmig um die Bodenfläche (5) der topfförmigen Elektrode (4) verläuft.

10. Meßwertaufnehmer nach einem der vorliegenden Ansprüche, dadurch gekennzeichnet, daß die Meßelektrode (7) Kathode und die Referenzelektrode (4) Anode einer polarographischen Meßzelle ist.


## Claims

1. A measured value pick-up in particular for the determination of the partial pressure of dissolved gases, e. g. a transcutaneous oxygen electrode, consisting of a carrier member (1) for at least one noble metal wire serving as a measuring electrode (7) and an annular reference electrode (4), the measuring electrode (7) being arranged in the opening (6) of the annular reference electrode (4), and for an element (8) for heating the measured value pick-up, the element (8) at the same time fulfilling the function of a heater and of a temperature sensor, characterised in that the annular reference electrode (4) is of a special pot-shape with the opening (6) in the base of the pot, the element (8) for heating the measured value receiver is directly arranged on the base of the pot-shaped reference electrode (4) around the opening (6).

2. A measured value pick-up as claimed in Claim 1, characterised in that the pot-shaped reference electrode (4) has a circular base (5) with a central opening (6).

3. A measured value pick-up as claimed in Claim 1, characterised in that the element (8) is a temperature-dependent resistance path.

4. A measured value pick-up as claimed in Claim 3, characterised in that the resistance path has an electric resistance in the order of magnitude of 100 ohm.

5. A measured value pick-up as claimed in Claim 3 or Claim 4, characterised in that the resistance path is formed by a flat coil made of thin wire, for example, copper, nickel or platinum.

6. A measured value pick-up as claimed in Claim 3 or Claim 4, characterised in that the resistance path is formed by resistance layers (31) applied to a substrate (30).

7. A measured value pick-up claimed in Claim 1, characterised in that the carrier member (1) has a supporting surface (3) for an adhesive mounting (14) which serves to fix the measured value receiver on the skin (20) of the measuring position.

8. A measured value pick-up as claimed in Claim 1, characterised in that the outer side of the base (5) of the pot-shaped reference electrode (4) is designed to be convex to the supporting surface (3) of the carrier member (1).

9. A measured value pick-up as claimed in Claim 8, characterised in that the supporting surface (3) runs annularly around the base (5) of the pot-shaped electrode (4).

10. A measured value pick-up as claimed in one of the previous Claims, characterised in that the measuring electrode (7) is the cathode and the reference electrode (4) is the anode of a polarographic measuring cell.


## Revendications

1. Détecteur de caleur de mesure, notamment pour la détermination de la pression partielle de gaz dissous, par exemple électrode à oxygène pour la voie percutanée, constituée d'une partie (1) formant support pour au moins un fil en métal précieux servant d'électrode de mesure (7) et pour une électrode annulaire de référence (4), l'électrode de mesure (7) se trouvant dans l'ouverture (6) de l'électrode annulaire de référence (4), ainsi que pour un élément (8) destiné à chauffer le détecteur de valeur de mesure, cet élément (8) remplissant à la fois la fonction d'un dispositif de chauffage et d'une sonde thermométrique, caractérisé en ce que l'électrode annulaire de référence (4) a la forme particulière d'un pot avec l'ouverture (6) ménagée dans le fond du pot, l'élément (8) pour chauffer le détecteur de valeur de mesure étant disposé autor de l'ouverture (6), directement sur le fond de l'électrode de référence en forme de pot.

2. Détecteur de valeur de mesure suivant la revendication 1, caractérisé en ce que l'électrode de référence (4) en forme de pot présente une surface de base (5) circulaire ayant une ouverture (6) centrale.

3. Détecteur de valeur de mesure suivant la revendication 1, caractérisé en ce que l'élément (8) a une voie résistante qui dépend de la température.

4. Détecteur de valeur de mesure suivant la revendication 3, caractérisé e ce que la résistance a une valeur de l'ordre de grandeur de 100 ohms.

5. Détecteur de valeur de mesure suivant la revendication 3 ou 4, caractérisé en ce que la résistance est constituée d'une bobine plate en fil mince, par exemple en cuivre, en nickel, ou en platine.

6. Détecteur de valeur de mesure suivant la revendication 3 ou 4, caractérisé en ce que la voie résistante est constituée de résistances (31) à couches déposées sur un substrat (30).

7. Détecteur de valeur de mesure suivant la revendication 1, caractérisé en ce que la partie (1) formant support présente une surface d'appui (31) pour une fixation par collage (14) destinée à la fixation du détecteur de valeur de mesure sur la peau (20), à l'endroit où la mesure doit être effectuée.

8. Détecteur de valeur de mesure suivant la revendication 1, caractérisé en ce que le fond (5) extérieur de l'électrode de référence (4) en forme de pot tourne sa convexité vers la surface d'appui (3) de la partie (1) formant support.

9. Détecteur de valeur de mesure suivant la revendication 8, caractérisé en ce que la surface d'appui (3) s'étend annulairement autour de la surface (5) du fond de l'électrode (4) en forme de pot.

10. Détecteur de mesure suivant l'une des revendications précédentes, caractérisé en ce que l'électrode de mesure (7) est la cathode et l'électrode de référence (4) est l'anode d'une cellule de mesure polarographique.

0 010 136

FIG 1

FIG 2

FIG 3

FIG 4

7